# EUROPEAN PATENT SPECIFICATION

(11) **EP 1 943 336 B1**
(45) Date of publication and mention of the grant of the patent: **30.04.2014**
(21) Application number: 06790851.7
(22) Date of filing: 13.10.2006
(51) Int. Cl.: C12N 5/0775, A61K 48/00, A61K 35/12, A61L 27/14, A61L 27/60, C12N 15/09, C12P 21/00, A61L 27/38

(54) **RECONSTRUCTED LIVING ADIPOSE TISSUE**
REKONSTRUIERTES LEBENDES FETTGEWEBE
TISSUS VIVANTS D'ADIPOSE RECONSTITUES

(30) Priority: 14.10.2005 US 726169 P
(43) Date of publication of application: 16.07.2008
(73) Proprietor: Université Laval, Quebec, QC G1V 0A6 (CA)
(72) Inventor: FRADETTE, Julie, Saint-Lambert-de-Lauzon, QUEBEC G0S SW0 (CA); GERMAIN, Lucie, Québec, Québec G1S 3C6 (CA); AUGER, François A., Québec, Québec G1S 1A9 (CA)
(74) Representative: Cabinet Plasseraud
(86) International application number: PCT/CA2006/001697
(87) International publication number: WO 2007/041869

(56) References cited:
- WO-A2-2004/007699
- US-A- 6 153 432
- US-A1- 2004 092 011
- HALVORSEN Y -D C ET AL: "Extracellular matrix mineralization and osteoblast gene expression by human adipose tissue-derived stromal cells" TISSUE ENGINEERING 2001 US, vol. 7, no. 6, 2001, pages 729-741, XP002510902 ISSN: 1076-3279
- GIMBLE J M ET AL: "Adipose-derived adult stem cells: Isolation, characterization, and differentiation potential" CYTOTHERAPY 2003 GB, vol. 5, no. 5, 2003, pages 362-369, XP009034648 ISSN: 1465-3249
- MICHEL M ET AL: "Characterization of a new tissue-engineered human skin equivalent with hair" IN VITRO CELLULAR AND DEVELOPMENTAL BIOLOGY - ANIMAL 199906 US, vol. 35, no. 6, June 1999 (1999-06), pages 318-326, XP008067234 ISSN: 1071-2690
- VERMETTE M ET AL: "Production of a new tissue-engineered adipose substitute from human adipose-derived stromal cells" BIOMATERIALS 2007 GB, vol. 28, no. 18, 2007, pages 2850-2860, XP022004006 ISSN: 0142-9612
- TORII S.I. ET AL: 'Ascorbic acid-2-phosphate enhances adipocyte differentiation of cultured stromal vascular cells prepared from bovine perirenal adipose tissue' ANIMAL SCIENCE AND TECHNOLOGY vol. 69, no. 5, 1998, pages 439 - 444
- VON HEIMBURG D. ET AL: 'Human preadipocytes seeded on freeze-dried collagen scaffolds investigated in vitro and in vivo' BIOMATERIALS vol. 22, no. 5, 2001, pages 429 - 438, XP003010983
- VON HEIMBURG D.: 'Influence of Different Biodegradable Carriers on the in Vivo Behavior of Human Adipose Precursor Cells.' PLASTIC AND RECONSTRUCTIVE SURGERY vol. 108, no. 2, 2001, pages 411 - 420, XP009032694
- GRENIER G. ET AL: 'Tissue Reorganization in Response to Mechanical Load Increases Functionality' TISSUE ENGINERING vol. 11, no. 1/2, 2005, pages 90 - 100, XP003010984

## Description

### FIELD OF THE INVENTION

The present invention relates to methods of producing tissues and tissue sheets assembled from adispose-derived stromal cells and methods of using such tissues and tissue sheets for pharmacological and toxicological studies as well as for grafts and/or implants.

### BACKGROUND OF THE INVENTION

Adipose tissue is a specialized connective tissue that functions as the major storage site of fat in the form of triglycerides. Adipose tissue is found in mammals in two different forms: white adipose tissue and brown adipose tissue. Besides its fat-storing function, the adipose tissue plays a crucial role in the regulation of body temperature, the resistance to mechanical impact as well as an endocrine function for modulating the physiology of the whole body.

Loss of adipose tissue can have a significant impact on quality of life issues in afflicted subjects. For example, facial acne in teenagers can result in the loss of subcutaneous adipose tissue and severe scarring. Similar soft tissue scarring and defects can also occur in cancer patients submitted to radiation therapy and major burn victims. Surgically-induced loss of adipose or soft tissue, such as the one experienced by cancer patients wherein a tumor has been surgically removed (e.g. patients who underwent a mastectomy for example), can also lead to severe scarring and defects. Lipodystrophy, a congenital or acquired condition, can also lead to the loss of adipose tissue. For example, AIDS patients receiving the tri-therapy drug treatment are more susceptible to develop a specific form of lipodystrophy. Surprisingly, severe loss of adipose tissue has metabolic effects similar to obesity such as insulin resistance and ultimately leads to the development of diabetes.

The injection of material for the regeneration of soft tissue began in 1899 when Gersuny injected paraffin for cosmetic purposes. Nowadays, the demand for soft tissue substitutes in reconstructive and plastic surgery is continually increasing. Unfortunately, the current autograft techniques fail to produce long-term satisfactory replacement [Patrick CW, Jr. Anat Rec 2001;263(4):361-6.]. This is due in part to the fragility of adipose tissue and the lack of appropriate vascularization after grafting [Billings E, Jr., May JW, Jr. Plast Reconstr Surg 1989;83(2):368-81.; Rohrich RJ, Sorokin ES, Brown SA. Plast Reconstr Surg 2004;113(1):391-5]. Efforts were made in the past to improve this situation, with the incorporation of dextran beads adsorbed with basic fibroblast growth factor [Eppley B L, Sidner R A, Platis J M, Sadove A M Plast Reconstruct Surg 90:1022-1030, 1992].

Tissue-engineering strategies are very promising as an alternative therapeutic approach to address the low predictability of autologous fat transplantation. Several groups have pioneered adipose tissue engineering using the commonly used 3T3-L1 preadipocyte cell line [Fischbach C.et al. Tissue Eng 2004;10(1-2):215-29; Patrick CW et al. Tissue Eng 2002;8(2):283-93; Patrick CW et al. Tissue Eng 1999;5(2):139-51] or bone-marrow derived mesenchymal stem cells [Alhadlaq A et al. Tissue Eng 2005;11(3-4):556-66]. The recent surge of interest in adipose tissue as a source of adult multipotent stem cells (ASCs) also generated encouraging results for their use in soft-tissue reconstruction [von Heimburg D et al. Biomaterials 2001;22(5):429-38; von Heimburg D et al. Plast Reconstr Surg 2001;108(2):411-20; U.S. Patent 6,777,231 issued on August 17, 2004; U.S. patent application 10/406479 published under 2004/0092011 on May 13, 2004].

Various scaffolding biomaterials have been tested in combination with these progenitor cells. Collagen gels, hyaluronic acid-based scaffolds [Halbleib M et al. Biomaterials 2003;24(18):3125-32], alginate beads [Marler JJ et al. Plast Reconstr Surg 2000;105(6):2049-58.], PLGA (poly(lactic-co-glycolic acid)) [Patrick CW et al. Tissue Eng 1999;5(2):139-51], PTFE (polytetrafluoroethylene) meshes coated with collagen and alginate or hyaluronic acid based hydrogels which can be dehydrated and rehydrated to obtain the desired shape [Alhadlaq A et al. Tissue Eng 2005;11 (3-4):556-66.]. Despite these efforts, tissue-engineered substitutes have not made it into the clinical realm yet, indicating the need to optimize and/or innovate with novel adipose engineering strategies.

Although there have been advancements, there were no successful generation of implantable reconstructed adipose or soft tissue in humans. One of the major limitations has been the failure to develop optimal conditions for the proliferation, expansion, and differentiation of recursors into three-dimensional tissue constructs *ex vivo*, the development of optimal conditions for the successful transplantation of autologous or allogeneic adipocytes such as the adequate vascularization of the transplanted tissues.

Considering the state of the art described above, there is a need for new soft tissue alternatives.

### SUMMARY OF THE INVENTION

The present invention relates to methods of producing tissues reconstructed from adipose-derived stromal cells (e.g. human adipose-derived stromal cells) and methods of using such tissues.

According to a first aspect, there is provided a method of producing a manipulatable adipose-derived tissue sheet, the method comprising contacting isolated adipose-derived stromal cells with a first medium comprising ascorbic acid, thereby producing the adipose-derived tissue sheet. In an embodiment, the concentration of ascorbic acid in the first medium is from about 20 to about 200 µg/ ml. In another embodiment, the concentration of ascorbic acid in the first medium is about 50 µg/ ml. In yet a further embodiment, the isolated adipose-derived stromal cells are derived from lipoaspirated fat and/or from excised fat. In still another embodiment, the method comprises further contacting the isolated adipose-derived stromal cells with a second medium comprising an adipogenic stimulus. In an embodiment, second medium comprises insulin, T3, dexamethasone, IBMX and a peroxisome proliferator-activated receptor gamma (PPARγ) agonist, and in a further embodiment, the PPARγ agonist is rosiglitazone and/or pioglitazone. In an embodiment, the isolated adipose-derived stromal cells are contacted simultaneously with the first medium and with the second medium. In a further embodiment, the isolated adipose-derived stromal cells are first contacted with the first medium and then with the second medium. In yet another embodiment, the isolated adipose-derived stromal cells are first contacted with the second medium and then with the first medium. In still another embodiment, the adipose-derived tissue sheet comprises adipose-derived stromal cells, and in a further embodiment, the concentration of adipose-derived stromal cells in the adipose-derived tissue sheet is about 100%. In still another embodiment, the adipose-derived tissue sheet comprises preadipocytes. In yet another embodiment, the adipose-derived tissue sheet comprises adipocytes, and in a further embodiment, the concentration of adipocytes in the adipose-derived tissue sheet is between about 20 to 90%. In yet another embodiment, the adipose-derived tissue sheet comprises cells (such as mammalian cells and/or human cells) and an extracellular matrix. In yet another embodiment, the extracellular matrix of the adipose-derived tissue sheet is produced (e.g. exclusively or partially) by the adipose-derived stromal cells. In still another embodiment, the method further comprises genetically modifying the adipose-derived stromal cells. In an embodiment, the genetic modification of the adipose-derived stromal cells is performed prior to contacting the adipose-derived stromal cells with said first medium. In still another embodiment, the genetic modification of the adipose-derived stromal cells is performed simultaneously to contacting the adipose-derived stromal cells with said first medium. In a further embodiment, the genetic modification of the adipose-derived stromal cells is performed after contacting the adipose-derived stromal cells with said first medium. In an embodiment, the genetic modification of the adipose-derived stromal cells is performed prior to contacting the adipose-derived stromal cells with said second medium. In still another embodiment, the genetic modification of the adipose-derived stromal cells is performed simultaneously to contacting the adipose-derived stromal cells with said second medium. In a further embodiment, the genetic modification of the adipose-derived stromal cells is performed after contacting the adipose-derived stromal cells with said second medium. In still another embodiment, the method further comprises adding a further cell type to the adipose-derived tissue sheet, and in a further embodiment, the further cell type is at least one of an endothelial cell type, an epithelial cell type, a fibroblastic cell type, a muscular cell type and a neuronal cell type.

There is provided an adipose-derived tissue sheet produced by the method described herein. For example, the adipose-derived tissue sheet has a thickness of between about 20 to 60 µm. The adipose-derived tissue sheet may produce a pro-angiogenic growth factor such as VEGF, angiopoietin-1 and/or FGF. The adipose-derived tissue sheet can produce an adipokine such as leptin and/or adiponectin. The adipose-derived tissue sheet may release glycerol and fatty acids in response to a lipolytic stimuli.

According to a further aspect, there is provided a method of producing a reconstructed conjunctive tissue, said method comprising superimposing at least two adipose-derived tissue sheets obtained by the method described herein, thereby producing the reconstructed conjunctive tissue. According to another aspect, there is provided a reconstructed conjunctive tissue produced by the method described herein. In an embodiment, the reconstructed conjunctive tissue has a thickness of between about 40 to 60 µm.

According to still a further aspect, there is provided a method of producing a reconstructed adipose tissue, said method comprising superimposing at least two adipose-derived tissue sheets produced by the method described herein, thereby producing the reconstructed adipose tissue. According to a further aspect, there is provided a reconstructed adipose tissue produced by the method described herein. In an embodiment, the reconstructed adipose tissue of has a thickness of between about 40 to 60 µm.

According to another aspect, there is provided a method of determining the adipocyte-modulating properties of an agent, said method comprising (i) producing a manipulatable adispose-derived tissue sheet described herein according to the method described herein, or producing an adispose tissue described herein according to the method described herein, (ii) contacting said agent with said tissue described herein and the reconstructed adipose tissue described herein and (iii) determining if said contact modulates a parameter of said tissue, thereby indicating the adipocyte-modulating properties of said agent. In an embodiment, the parameter is at least one of the number of cells in the tissue, the size of the cells in the tissue, the roundness of the cells in the tissue, the degree of differentiation of the cells in the tissue, the quantity of lipids in the cells of the tissue, the composition of lipids in the cells of the tissue, the ability of the cells of the tissue to produce an adipokine, the ability of the cells of the tissue to produce a pro-angiogenic growth factor, the ability of the cells of the tissue to respond to a lipolytic stimuli, the ability of the cells of the tissue to replicate, the viability of the cells of the tissue and the ability of the cells of the issue to express a gene related to an adipocyte metabolic function (such as FOXC2, PGC-1, UCP-1, GATA2, PPARα and/or PPARγ).

There is also provided a method of remodeling a body part in a subject in need thereof, said method comprising introducing into said subject a tissue being at least one of the adipose-derived tissue sheet described herein, the reconstructed conjunctive tissue described herein and the reconstructed adipose tissue described herein, thereby remodeling said body part of said subject. In an embodiment, the body part comprises a cavity filled by said tissue, and in a further embodiment, the cavity is filled partially with said tissue. In a further embodiment, the cavity is associated with at least one of a burn, a surgery, an hereditary condition, a trauma and the intake of a therapeutic agent. In a further embodiment, the size of said body part is increased by the introduction of said tissue. In this particular embodiment, the body part may be at least one of a lip, a breast, a buttock, a chin, a cheek, an upper body and a thigh. In still another embodiment, the subject is a human. In yet another embodiment, the tissue used can comprises genetically modified cells and/or cells autologous to the subject.

There is further provided the use of a tissue being at least one of the adipose-derived tissue sheet described herein, the reconstructed conjunctive tissue described herein, the reconstructed adipose tissue for remodeling a body part in a subject. In an embodiment, the tissue is adapted to fill a cavity in the body part (wholly or partially). In a further embodiment, the cavity of the body part is associated with at least one of a burn, a surgery, an hereditary condition, a trauma and the intake of a therapeutic agent. In still another embodiment, the tissue is adapted to increase the size of the body part. In this particular embodiment, the body part can be at least one of a lip, a breast, a buttock, a chin, a cheek, an upper body and a thigh. In a further embodiment, the subject is a human. In yet another embodiment, the tissue can comprise genetically modified cells and/or cells autologous to said subject.

### BRIEF DESCRIPTION OF THE FIGURES

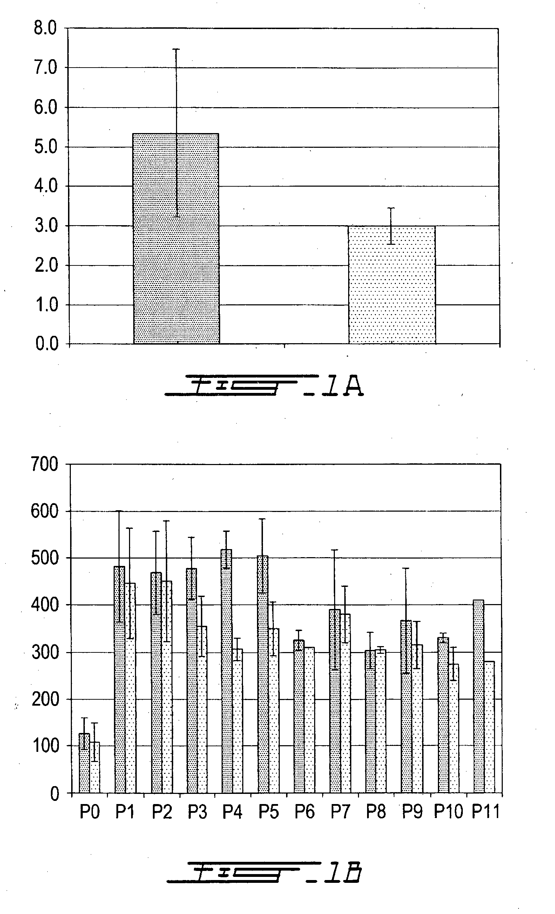

Figure 1. Human subcutaneous adipose tissue as a source of precursor cells for tissue engineering purposes. (**A**) Cell yield at the extraction after collagenase digestion were higher for lipoaspirated fat (LA)-derived stromal cells (grey box) compared to lipectomy-derived (LP) cells from excised fat (dotted box). Results are expressed as means in 10⁵ of cells per gram of tissue (for LA 11 extractions from three different tissues, for LP 6 extractions from three different tissues). (**B**) Number of cells obtained for LA-derived stromal cells (grey box) and LP-derived stromal cells (dotted box) between passage 0 to 11 (P0 to P11). Results are expressed as means in 10³ cells per cm² per day.

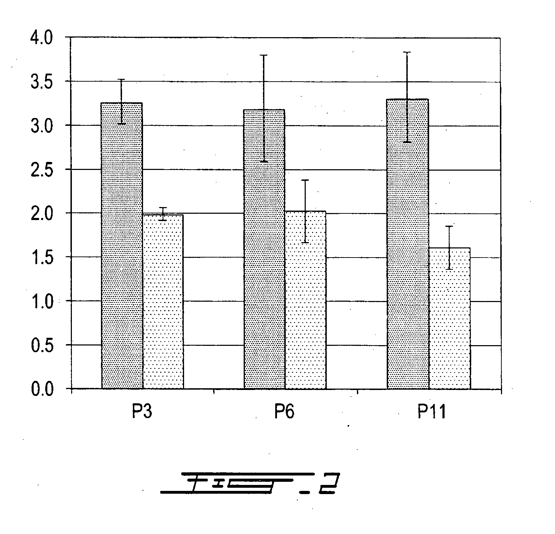

Figure 2. Adipogenic potential of stromal cells extracted from lipoaspirated fat (LA) (grey box) compared to excised fat (LP) (dotted box). Cells were cultured for 7 days in the presence of ascorbic acid and prior to the induction of differentiation, which was maintained for 14 days with the adipogenic cocktail and ascorbic acid before oil red-O (ORO) staining (OD 520 nm). The Y axis refers to the OD ratio measurement for the ORO staining, the X axis refers to the number of passage of the cells (P3, P6 or P11). (n=3 for LA, n=3 for LP with 2-6 replicates per cell line per passage)

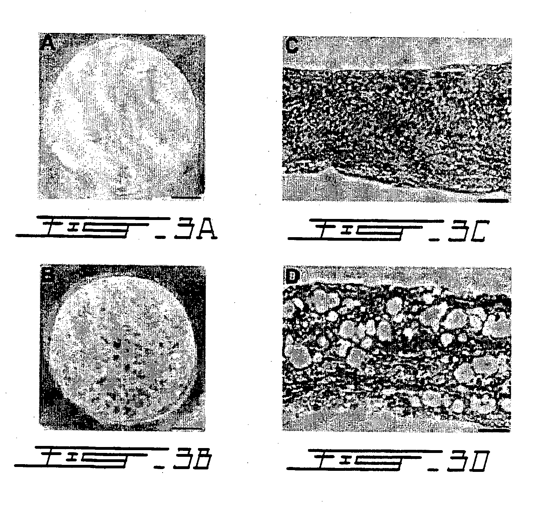

Figure 3. (**A-B**) Representative macroscopic view and (**C-D**) histological appearance (cross sections) of tissues reconstructed from adipose-derived stromal cells using the technique described in Example I. (**A**, **C**) Stromal cells that were cultivated in presence of ascorbic acid for 37 days, without inducing the differentiation into adipocytes, produced a conjunctive-like stroma, while (**B, D**) stromal cells submitted to an adipogenic cocktail on day 7 of culture generated a tissue filled with adipocytes, as shown after ORO staining (**B**) and (**D**) Masson's trichrome staining of paraffin-embedded tissue sections. Bars : (**A-B**) 16.5 mm, (**C-D**) 36.3 µm.

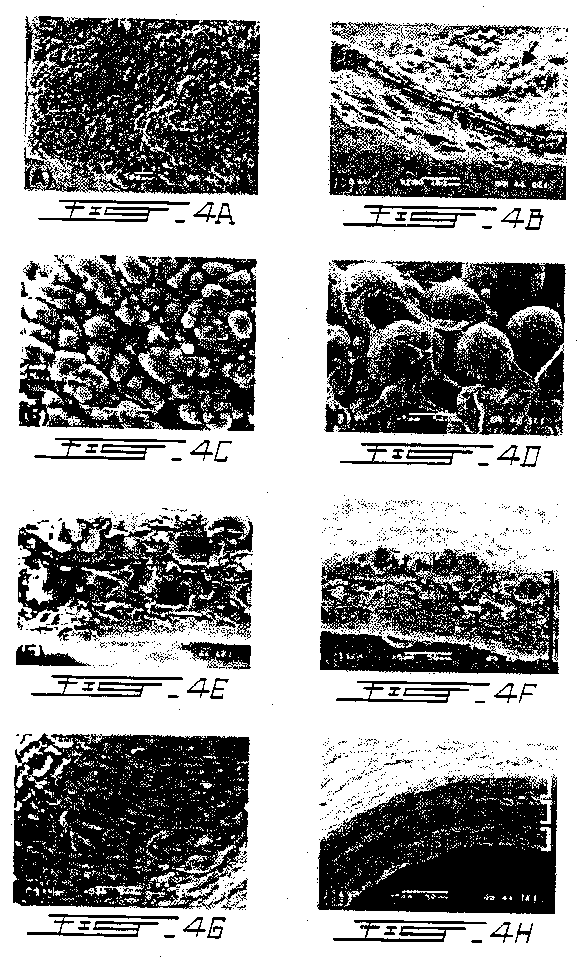

Figure 4. Representative microarchitecture of the reconstructed adipose tissue by scanning electron microscopy (SEM). (**A-B**) Low magnification micrographs showing the surface appearance of the reconstruction adipose tissue, revealing the presence of round adipocytes on both sides of the tissue (arrows, **B**). (**C**) After 42 days of *in vitro* differentiation, the adipocytes of the reconstructed adipose tissue are very similar to those of human subcutaneaous adipose tissue (**D**). (**E**-**F**) Cross-section micrographs of a reconstructed adipose tissue clearly show the round adipocytes embedded within each of the three adipose sheets that were combined to form a cohesive thicker tissue (brackets, **F**), while reconstructed conjunctive tissue is devoid of adipocytes (**H**). (**G**) The surface of the reconstructed conjunctive tissue produced by adipose-derived stromal cells which were not induced to differenciate into adipocytes shows a dense mesh-like tissue made of flat cells and extracellular matrix. (**A-D, G**) are SEM hexamethydisilazan-treated specimens while (**E, F, H**) are CO₂ critical point-treated specimens. Bars : (**A**, **B**) 100 µm, (**C, D, F, G, H**) 50 µm and (**E**) 20 µm.

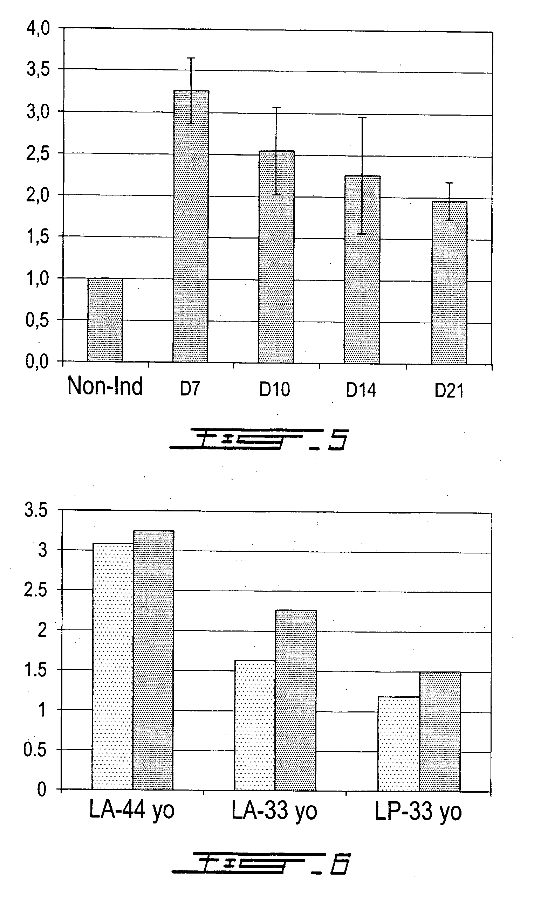

Figure 5. Adipocyte differentiation within adipose sheets produced from stromal cells as a function of the day of differentiation. Cells were cultured in presence of ascorbic acid for the entire length of the experiment. After 7, 10, 14 or 21 days of culture respectively, differentiation was induced by adding an adipogenic cocktail, followed by 14 days of culture under adipogenic conditions. Differentiation was measured using ORO staining and spectrometric analysis. Non-Ind refers to control stromal cells not induced with the adipogenic cocktail. The Y axis refers to the OD ratio measurement for the ORO staining, the X axis refers to the day of culture at which induction of differentiation was performed. (n=3 for LA, 2-6 replicates per time point).
Figure 6. Promotion of adipocyte differentiation by ascorbic acid of stromal cells not submitted to freezing. Fresh cells from three different sources (LA of 44 year-old subject, LA of 33-year old subject and LP from 33 year-old subject) were cultured in a medium without ascorbic acid (dotted box) or with ascorbic acid (grey box). Adipocyte differentiation was then assessed using the ORO staining and spectrometric analysis. The Y axis refers to the OD ratio measurement for the ORO staining, the X axis refers to the three different cell types.

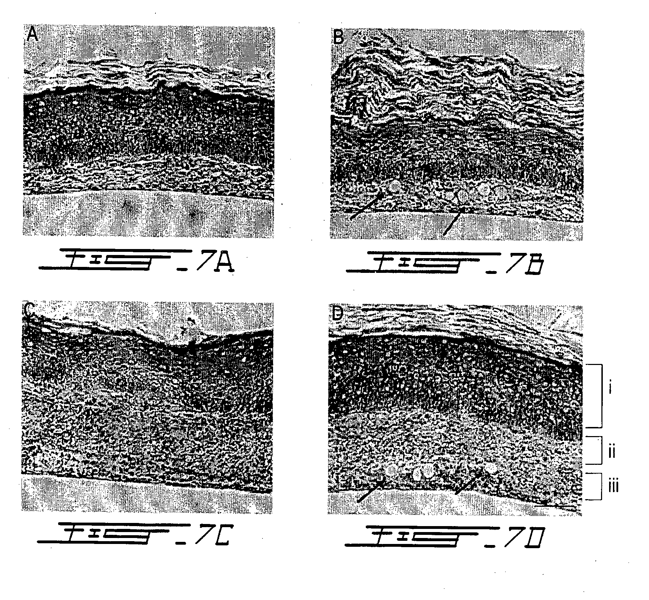

Figure 7. Histological cross-sections of reconstructed human skin tissues comprising adipose-derived stromal cells. (**A, B**) only keratinocytes and stromal cells have been used whereas (**C, D**) keratinocytes, dermal fibroblasts and stromal cells have been used. Adipose-derived stromal cells found in (**A, C**) have not been induced with the adipogenic cocktail whereas those found in (**B, D**) have been induced with the adipogenic cocktail. Arrows point to lipid droplet accumulation within an adipocyte. The letter "i" refers to the epidermis, "ii" to the dermis and "iii" to the hypodermis.

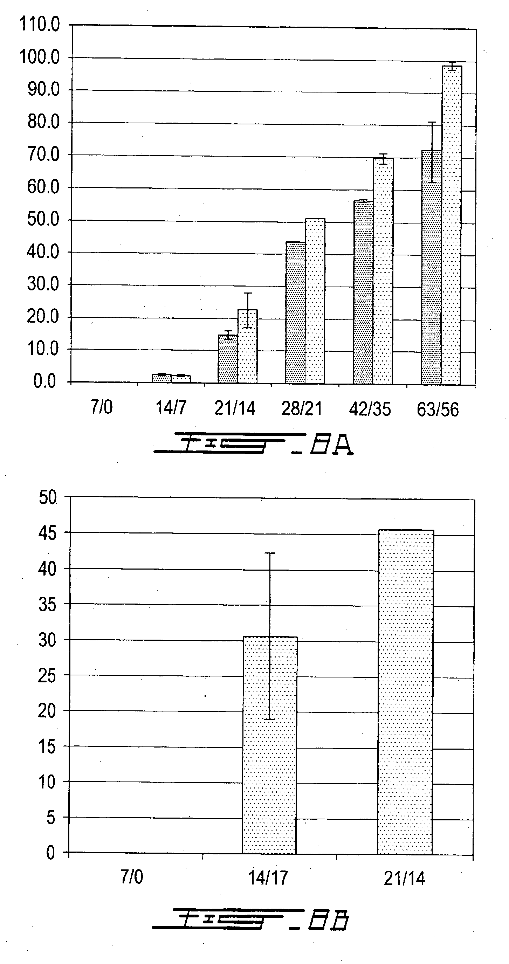

Figure 8. Tissue-engineered adipose sheets secrete high levels of the major adipokines (**A**) leptin and (**B**) adiponectin. Results are expressed as ng/ml/24h per adipose sheet of 3.5 cm². Adipokine concentrations were measured, at the indicated times, by specific ELISA in serum-free medium conditioned for 24h. Numbers under each boxes refer to the number of days in culture/the number of days submitted to an adipogenic cocktail. Data are given as mean ± SD (n=2 at each time point, for three different cell lines for each cell type).

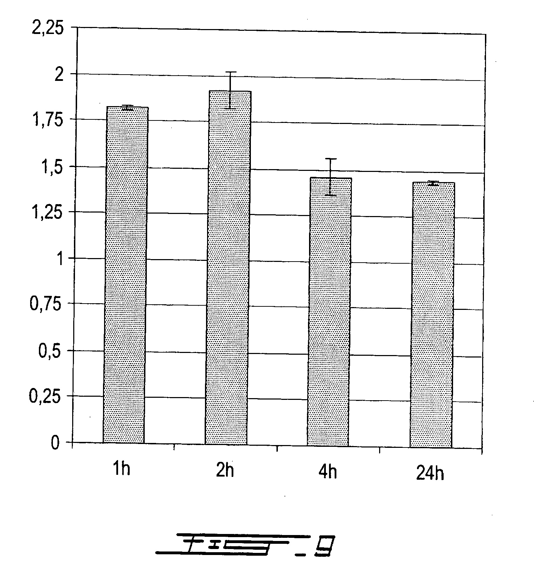

Figure 9. Tissue-engineered adipose sheets display lipolytic activation induced by adrenoceptor agonists. Isoproterenol (1 µM), a general agonist of β-adrenergic receptors, induced the hydrolysis of triclycetides stocked within adipocytes into fatty acids and glycerol. Glycerol release is quantified by a colorimetric assay and expressed as fold over basal levels observed for the vehicle treated samples. Basal levels were 16.1 ± 1.3 µM at 2h, 31.1 ± 1.0 µM at 4h and 240.5 ± 5.8 µM after 24h. Adipose constructs produced from lipoaspirated fat in P3 were assayed (n=4) after 20 days of *in vitro* differentiation.

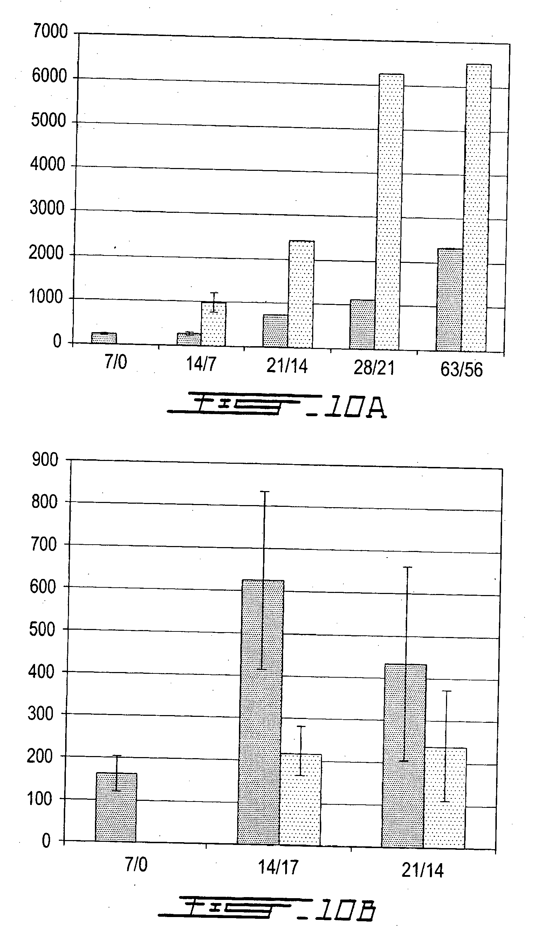

Figure 10. Tissue-engineered adipose-derived tissue sheets secrete pro-angiogenic growth factors (**A**) Angiopoietin-1 (Ang-1) and (**B**) VEGF. Results are expressed as pg/ml/24h per adipose sheet of 3.5 cm². (A) Ang-1 secretion profiles in adipose-derived tissue (stromal undifferentiated cells, grey box) or in adipose tissue sheets (differentiated adipocytes, dotted box) (n=2 cell populations). (**B**) VEGF secretion profiles in adipose-derived tissue (stromal undifferentiated cells, grey box) or in adipose tissue sheets (differentiated adipocytes, dotted box) (n=5 cell populations). Numbers under each boxes refer to the number of days in culture/the number of days submitted to an adipogenic cocktail.

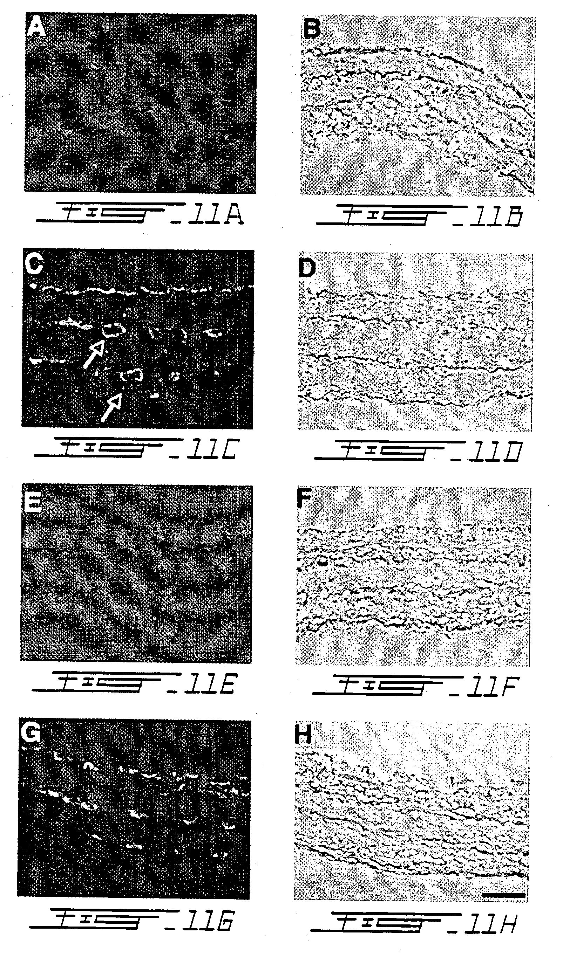

Figure 11. Formation of pseudo-capillaries *in vitro* within human reconstructed adipose tissue. PECAM specific staining is shown in A, C, E and G whereas corresponding phase contrast are shown in B, D, F and H. (**A, B**) reconstructed adipose tissue without endothelial cells, (**C, D**) reconstructed adipose tissue to which endothelial cells have been added (arrows in C point to PECAM positive tubular structures) (**E, F**). Reconstructed conjunctive tissue without endothelial cells, (**G,H**) to which endothelial cells have been added. Bar : 100 µm.

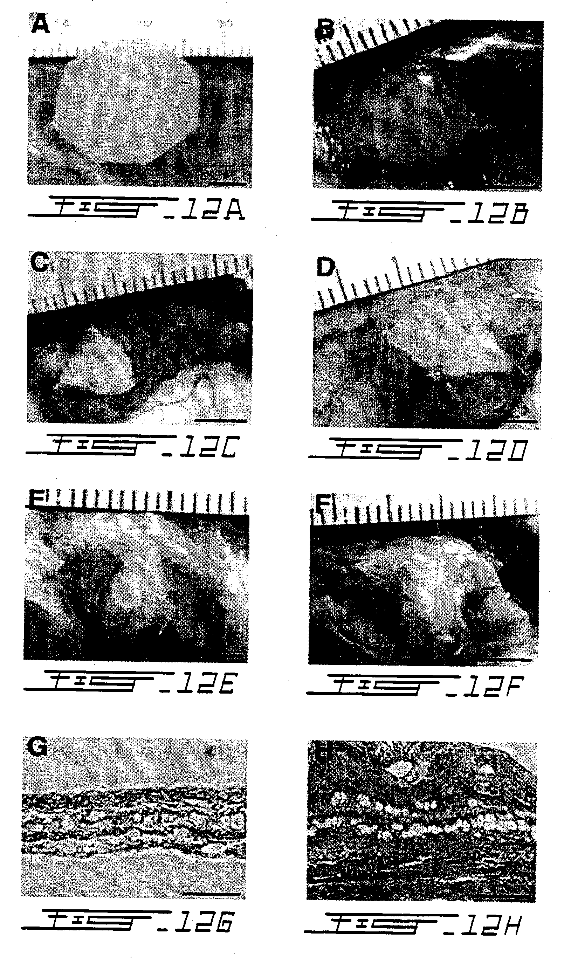

Figure 12. *In vivo* implantation of reconstructed adipose tissue in athymic mice. Reconstructed adipose tissue were grafted onto the muscle on the back of mice. (A) Macroscopic aspect of the tissue before implantation, (**B**) 3 days, (**C-D**) 7 days and (**E-F**) 14 days after the surgery. (**G-H**) Masson's trichrome staining showing histological features of the tissue (**G**) before and (**H**) 7 days following implantation showing a good implantation of the tissue and the viability of adipocytes within the reconstructed tissues. Bars: (**A-F**) 5 mm, (**G**) 100 µm, (**H**) 200 µm.

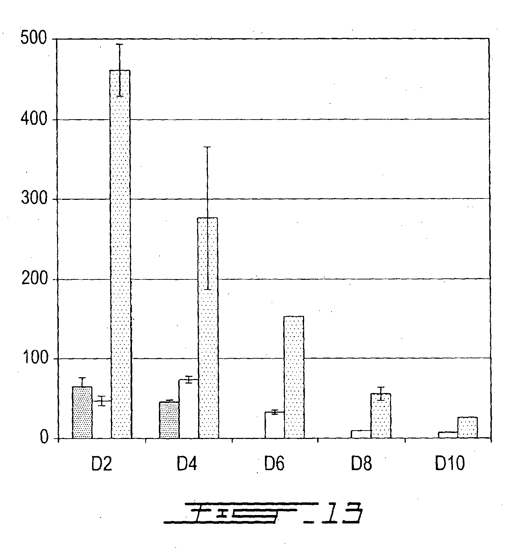

Figure 13. Genetic modification of adipose-derived stromal cells with a viral vector harboring a recombinant factor IX transgene. Transgene expression was measured 2, 4, 6, 8 and 10 days after the initial transduction of preadipocytes (grey box), induced preadipocytes (white box) and mature adipocytes differentiated for 20 days (dotted box). Results are expressed as ng per mg protein per 10⁶ cells (n=2).

### DETAILED DESCRIPTION OF THE PREFERRED EMBODIMENTS

The present invention relates to methods of producing tissue sheets and reconstructed tissues from adipose-derived stromal cells as well as methods of using such tissue sheets and reconstructed tissues.

Unless otherwise defined, all terms of art, notations and other scientific terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. The techniques and procedures described or referenced herein are generally well understood and commonly employed using conventional methodology by those skilled in the art, such as, for example, the widely utilized molecular cloning methodologies described in Sambrook et al., Molecular Cloning: A Laboratory Manual 2nd. edition (1989) Cold Spring Harbor Laboratory Press, Cold Spring Harbor, N.Y. As appropriate, procedures involving the use of commercially available kits and reagents are generally carried out in accordance with manufacturer defined protocols and/or parameters unless otherwise noted.

According to one aspect, the present invention provides a method of producing an adipose-derived tissue sheet. In order to obtain such tissue sheet, the method comprises contacting isolated adipose-derived stromal cells with ascorbic acid. It is to be understood that the production of tissue sheets is to be performed *in vitro* or *ex vivo* and excludes the production of adipose-derived tissue sheets *in vivo.*

As used herein, the term "adipose-derived stromal cells" are cells obtained from the stromal portion of fat. The adipose-derived stromal cells are adherent cells that can proliferate *in vitro* and that are capable of differentiating into mature adipocytes when submitted to an appropriate adipogenic stimuli. The adipose-derived stromal cells are not adipocytes *per se* but precursors of adipocytes. The adipose-derived stromal cells can contain preadipocytes.

Surprisingly, it was found that when adipose-derived stromal cells are contacted with ascorbic acid, they produce an extracellular matrix in which they embedded themselves. The production of extracellular matrix components can easily be observed when cells have reached confluency. Even more surprisingly, after at least 21 or 28 days of culture, the adipose-derived stromal cells and the extracellular matrix they have produced can easily be handled by a person skilled in the art. As used herein, the term "adipose-derived tissue sheet" refer to a manipulatable tissue sheet assembled by adipose-derived stromal cells, free of any exogenous synthetic material.

Because adipose-derived stromal cells are adherent cells cultured in the presence of a liquid medium, ascorbic acid is preferably added to the culture medium of the cells. In an embodiment, the culture medium of the adipose-derived stromal cells is changed every 2 to 3 days and replaced with a fresh culture medium comprising the ascorbic acid. In a further embodiment, the fresh culture medium of adipose-derived stromal cells can be supplemented with ascorbic acid the entire length of the *in vitro* culture of the adipose-derived tissue sheet or only for a fraction of the *in vitro* culture. Preferably, the fresh culture medium is supplemented with ascorbic acid until the adipose-derived stromal cells form an adipose-derived tissue sheet that can be easily handled. In an embodiment, the concentration of ascorbic acid in the fresh culture medium is from about 20 to about 200 µg/ml. In another embodiment, the concentration of ascorbic acid in the fresh culture medium is about 50 µg/ml.

The adipose-derived stromal cells can be obtained by various techniques known to those skilled in the art. The source of adipose-derived stromal cells are numerous and include subcutaneous, muscular and/or visceral fat. In an embodiment, the fat can be obtained from a surgical procedure such as liposuction or any other surgical procedure where fat is removed from a subject and wherein viable adipose-derived cells can be obtained. In the present application, the liposuction procedure was compared to lipectomy (excision of fat). The liposuction and the lipectomy procedures have generated fat tissues comprising viable adipose-derived stromal cells. Once plated, the adipose-derived stromal cells can be submitted directly to the method described herein to produce adipose-derived tissue sheets or they can be first expanded *in vitro*, optionally frozen for future use, and then submitted to the method described herein.

Once isolated from fat and cultured *in vitro*, the adipose-derived stromal cells can be contacted with an adipogenic stimulus. The adipogenic stimulus is needed for the differentiation of adipose-derived stromal cells in adipocytes. As used herein, the term "preadipocyte" refers to cells capable of differentiating into adipocytes when submitted to an adipogenic stimulus and to cells who do not contain visible lipid droplets (e.g. lipid droplet-free cells). Although there is no consensus in the art, it has been suggested that non-differentiated preadipocytes express the Pref-1 and that more differentiated preadipocytes can express the adipocyte fatty acid binding protein (aP2), although the latter is mostly expressed in differentiated adipocytes. As used herein, the terms "adipocyte", "mature adipocyte" or "differentiated adipocyte" refer to cells capable of storing triglycerides as well as releasing glycerol and fatty acids when appropriately stimulated. Various adipogenic stimuli can be used to differentiate adipose-derived stromal cells to adipocytes. One adipogenic stimulus commonly used by those skilled in the art comprises a mixture of insulin, triiodothyronine (T3), dexamethasone, isobutylmethylxanthine (IBMX) and a peroxisome proliferator-activated receptor gamma (PPARγ) agonist (such as rosiglitazone and/or pioglitazone). It has been surprisingly shown that ascorbic acid can facilitate cellular differentiation of fresh (e.g. non-frozen) adipose-derived stromal cells into adipocytes. The adipose-derived stromal cells can be submitted to the adipogenic stimulus prior to, simultaneously, concomitantly or posterior to ascorbic acid. What has surprisingly been found is that adipose-derived stromal cells even though embedded in a thick and complex extracellular matrix of the adipose-derived tissue sheet remain sensible to the adipogenic stimulus can differentiate into mature adipocytes.

The adipose-derived tissue sheet produced by the method described herein can comprise cells, more particularly living cells. In an embodiment, the cells may be from a mammalian origin and, in a further embodiment, from a human origin. The cells of the adipose-derived tissue sheet may be adipose-derived stromal cells, preadipocytes and/or adipocytes. Since there are no specific markers for preadipocytes, it is not yet possible to quantify the number of these cells in the adipose-derived tissue sheet. The adipose-derived tissue sheet may comprise adipocytes, when the adipose-derived stromal cells have been submitted to an adipogenic stimulus. The concentration of adipocytes in the adipose-derived tissue sheet varies between about 20 to 90% of the total cells present in the adipose-derived tissue sheet.

In an embodiment, the method described herein may also comprise genetically modifying the adipose-derived stromal cells. This step may be particularly useful when the adipose-derived tissue sheet is intended to be grafted or implanted, then the genetically modified adipose-derived stromal cells can be used to express and produce a transgene that may be beneficial for the subject (e.g. increasing graft take, release of a drug or a pro-drug, etc.). The transgene that can be inserted may be, for example, a pro-angiogenic factor that will favor vascularization of the grafted or implanted adipose-derived tissue sheet. In an embodiment, the genetic modification step may be performed prior to, simultaneously or posterior to the contacting with the ascorbic acid. In a further embodiment, the genetic modification step may be performed prior to, simultaneously, concomitantly or posterior to the contacting with the adipogenic stimulus. In yet another embodiment, the genetic manipulation may comprise the introduction of a vector comprising a transgene in the adipose-derived stromal cells. The vector may be plasmid, a cosmid, an artificial chromosome and/or a viral-based vector. Various vectors can be used but those capable of being expressed in adipose-derived stromal cells are preferred. The transgene of interest may be, depending on the intended use, expressed constitutively or expressed upon induction only. Alternatively or optionally, the expression of the transgene may be transient or continuous. In an embodiment, a herpes simplex virus type 1-based vector, an adenovirus-based vector and/or a lentiviral vector is introduced in the adipose-derived stromal cells.

The adipose-derived tissue sheet produced by the method described herein comprises an extracellular matrix. As mentioned above, when the adipose-derived stromal cells reach confluency and are contacted with ascorbic acid, they start producing a rich and complex extracellular matrix in which they embed themselves. In an embodiment, the extracellular matrix present in the adipose-derived tissue sheet is exclusively produced by the adipose-derived stromal cells. In a further embodiment, when the adipose-derived stromal cells are human cells, the extracellular matrix is solely composed of human extracellular matrix components and is devoid of non-human extracellular matrix components. The extracellular matrix of the adipose-derived tissue sheet may comprise common matrix elements such as collagens (type I to type V), laminins, proteoglycans (such as decorin), glycosaminoglycans, versican, fibulin, elastin, fibronectin, tenascin, thrombospondin, etc. In addition, when adipose-derived tissue sheets are seeded with another cell type, such as endothelial cells or epithelial cells, the extracellular matrix it contains may also comprise matrix elements of the basal membrane (laminins, type IV collagen, nidogen, perlecan, etc.).

In a further embodiment, the method may also comprise adding a further cell type to the adipose-derived tissue sheet. The added cells may form organ-like structures in association with the adipose-tissue sheet. For example, the further cell type may be seeded, proliferate and differentiate at the surface of the adipose-derived tissue sheet (such as an epithelial cell type) or it may penetrate the matrix, proliferate and differentiate inside the adipose-derived tissue sheet (such as an endothelial cell type, a neuronal cell type and/or a muscular cell type). Various cell types may be added to the adipose-derived tissue sheets. These cell types include, but are not limited to, an endothelial cell type, an epithelial cell type, a fibroblastic cell type, a muscular cell type and a neuronal cell type. The further cell type can also include pluripotent stem cells, excluding human embryonic cells, non-differentiated cells and/or differentiated cells. In an embodiment, the adipose-derived tissue sheet may be used as an hypodermal substitute for reconstructed skin. In this particular embodiment, skin epithelial cells (keratinocytes) are placed directly or indirectly on the surface of at least one adipose-derived tissue sheet. A fibroblastic tissue sheet may optionally be placed between the epithelium and the adipose-derived tissue sheet. Endothelial cells can optionally be added to the fibroblastic tissue sheet and/or the adipose-derived tissue sheet to form a capillary network.

Since it has been shown herein that the adipose-derived tissue sheet can produce pro-angiogenic growth factors and sustain the growth and differentiation of endothelial cells into capillary-like structures, the adipose-derived tissue sheet may also serve as a matrix for the establishment of a three-dimensional capillary network. A complex network of small blood vessels are present in the hyperdermal portion of the skin and the adipose-derived tissue sheet described herein can serve as an *in vitro* model for this portion of the skin. The model can also be used to study capillary formation in this portion of the skin, to screen potential angiogenic and/or adipogenic modulating agents, and/or to study the effect of genetic modification of the adipose-derived cells on the establishment and maintenance of a capillary network.

The present invention also provides an adipose-derived tissue sheet obtained by the method described herein. The adipose-derived tissue sheet has numerous advantages. In an embodiment, the adipose-derived tissue sheet can be easily handled by those skilled in the art (such as laboratory technicians and surgeons). In a further embodiment, the adipose-derived tissue sheet has a thickness between about 20 to 60 µm depending on culture conditions (such as serum lot). In a further embodiment, the adipose-derived tissue sheet, when submitted to an adipogenic stimulus, is representative of a functional white adipose tissue, capable of triglyceride biosynthesis, secreting of typical adipokines and β-adrenergic stimulated lipolysis. For example, the adipose-derived tissue sheet is capable of expressing and secreting an adipokine (such as leptin and adiponectin), a pro-angiogenic growth factor (such as VEGF, angiopoietin-1 and/or FGF) and is sensitive to a lipolytic stimulus. In yet another embodiment, the adipose-derived tissue sheet consists essentially of cells derived from adipose-derived stromal cells and the extracellular matrix the adipose-derived cells have produced and is devoid of exogenous extracellular matrix components.

In a further aspect, the present invention also provides a method of producing a reconstructed adipose tissue, said method comprising superimposing at least two adipose-derived tissue sheets obtained by the method described herein. Because the reconstructed adipose tissue comprises adipocytes, the tissue or the tissue sheet must be submitted to an adipogenic stimulus to enable the differentiation of adipose-derived stromal cells into adipocytes. As mentioned above, the method can also comprise adding other cell types to the reconstructed adipose tissue to generate an organ-like tissue. In yet another aspect, the present invention also provides a reconstructed adipose tissue obtained by the method described herein. In an embodiment, thickness of such reconstructed adipose tissue is about 200 µm. Once dehydrated and prepared for histological studies, the thickness of the reconstructed adipose tissue is about 140 ± 14 µm.

In a yet another aspect, the present invention also provides a method of producing a reconstructed conjunctive tissue, said method comprising superimposing at least two adipose-derived tissue sheets obtained by the method described herein. Because the reconstructed conjunctive tissue does not comprise adipocytes, therefore, the tissue or the tissue sheet must not be submitted to an adipogenic stimulus to enable the differentiation of adipose-derived stromal cells into adipocytes. As mentioned above, the method can also comprise adding other cell types to the reconstructed conjunctive tissue to generate an organ-like tissue. For example and as shown below in the Examples, the reconstructed conjunctive tissue can serve as a stromal component capable of supporting the growth of epithelial cells such as keratinocytes.

Some therapeutic agents, especially combinations of therapeutic agents, are known to induce lipodystrophy in some subjects. It is thus desirable to have a screening method capable of assessing the adipocyte-modulating properties of a therapeutic agent. Since animal testing of cosmetic product is banned in various countries, it is also desirable to possess an *in vitro* toxicological screening method with a reconstructed skin having the three layers normally found in intact skin. Furthermore, since adipocyte metabolism and differentiation is not clearly understood yet, there is a need for an *in vitro* three-dimensional human adipogenesis model. Therefore, in still a further aspect, the present invention provides a method of determining the adipocyte-modulating properties of an agent. In this particular method, a tissue (such as the adipose-derived tissue sheet described herein, the reconstructed conjunctive tissue described herein and/or the reconstructed adipose tissue described herein) is contacted with the agent. Then, it is determined if such contact modulates a parameter of the tissue. For example, such parameter may be selected from the group consisting of the number of cells in the tissue, the size of the cells in the tissue, the roundness of the cells in the tissue, the differentiation of the cells in the tissue, the quantity of lipids in the cells of the tissue, the composition of lipids in the cells of the tissue, the ability of the cells of the tissue to produce an adipokine, the ability of the cells of the tissue to produce a pro-angiogenic growth factor, the ability of the cells of the tissue to respond to a lipolytic stimuli, the ability of the cells of the tissue to replicate or undergo apoptosis, the viability of the cells of the tissue and/or the expression of adipogenic-related metabolic genes of the cells of the tissue (such as FOXC2, PGC-1, UCP-1, GATA2, PPARα and/or PPARγ).

If such contacts modulate at least parameter, then the agent is said to have adipocyte-modulating properties. The method can also comprise comparing the parameter with a control tissue not exposed to the agent for assessing the modulation. When a cosmetic product is used as an agent in this method, it is preferable that the adipose-derived tissue sheet be placed underneath a fibroblastic tissue sheet and a differentiated epithelium to mimic closely the skin *in vivo* situation.

Further, because the adipose-derived tissue sheet, especially when it is assembled into a reconstructed adipose tissue, can mimic a white adipose tissue, it can be used to study the metabolism of the white adipose tissue, such as the conversion of white adipose tissue into brown adipose tissue.

Because the adipose-derived tissue sheet and the reconstructed adipose tissue recreate a soft tissue replacement alternative, the present invention also provides a method of remodeling a body part in a subject in need thereof. The method comprises introducing into said subject the adipose-derived tissue sheet, the reconstructed adipose tissue and/or the reconstructed conjunctive tissue produced by the methods described herein. In an embodiment, the body part comprises a cavity to be filled (partially or completely) by the adipose-derived tissue sheet and/or the reconstructed adipose tissue. In a further embodiment, the body part's cavity is associated with a burn (such as a third degree bum), a surgery, an hereditary condition, a trauma and/or the intake of a drug (such as a lipodystrophy-inducing drug). This method can be applied to any body part normally comprising adipose or soft tissues. For example, hips, lips, breasts, buttocks, chin, upper body part and/or the site of injection of a therapeutic agent (such as insulin) can be remodeled by this method. In another embodiment, the method can also be used to replaced the hypodermic portion of in deep burns (such as third degree burns). For cosmetic purposes, and in yet another embodiment, the size of the body part (such as the lips, the breast and/or the buttock) can be increased by the adipose-derived tissue sheet. The method can be used on various subjects, such as human subjects.

Also contemplated herein is the use of the adipose-derived tissue sheet produced by the method described herein for remodeling a body part in a subject as well as the use of the reconstructed living adipose tissue produced by the method described herein in the preparation of a dermal and/or hypodermal layer substitute.

This body remodeling technique can be advantageous, especially if one uses the patient's own cells to produce an adipose-derived tissue sheet or and reconstructed adipose tissue and reintroduces such adipose-derived tissue sheet or reconstructed adipose tissue into the same patient. This autologous treatment prevents or limits graft/implant rejection. Although this method can be used with autologous cells, it can also be used with heterologous cells or a combination of autologous and heterologous cells. The cells used in this method can also be genetically modified to enhance graft take or to deliver a therapeutic agent to the patient.

The present invention will be more readily understood by referring to the following examples which are given to illustrate the invention rather than to limit its scope.

### EXAMPLE I - Preparation of reconstructed adipose and conjunctive tissues

*Isolation and culture of stromal cells from human adipose tissue.* Cells were extracted from adipose tissues of healthy subjects undergoing cosmetic surgery procedures following guidelines from Laval University Ethic Board. Tissues were harvested either as excised fat from lipectomy biopsies (LP, n=3) or as lipoaspirated fat (LA, n=3) with a 4 mm cannula. In one case, LP and LA cells were obtained from the same patient (age 33). The mean age of donors was respectively 37.7 ± 9.9 years for LP and 39.0 ± 5.6 years (LA) The mean body-mass index (BMI) of donors was 23.5 ± 0.4 for LP and 23.6 ±2.9 (LA). The harvested tissues allowed to process approximately 4 times 60 grams of fat according to standard stromal cell extraction protocols [Hauner H et al, J Clin Invest 1989;89(5):1663-70., Zuk et al. Tissue Eng 2001;7(2):211-28]. Adipose tissues were digested with 0.075% collagenase (type 1A, Sigma) in Krebs-Ringer Buffer for 60 minutes at 37°C followed by a 10 min. treatment with 0.25% trypsin. Floating adipocytes were discarded and cells from the stromal-vascular fraction were pelleted, rinsed with media, centrifuged and a red cell lysis step in NH₄Cl was performed for 10 min. at room temperature. The cells recuperated are herein referred to as "adipose-derived stromal cells". To assess yield reproducibility, a total of 11 stromal cell extractions were performed from lipoaspirated fat (LA) and 6 extractions were performed from excised fat (LP). The cells obtained were seeded at a density of 8x10⁵ cells per cm² for expansion and batch cryopreservation after primary culture (P0). Part of the fresh stromal cells were fixed in 70% ethanol for flow cytometry (FACS). Experiments were performed on cells in P3, P6 and P11 that were thawed from P0 and expanded at a density of 8x10³ cells per cm² in DH 1:1 medium supplemented with 10% FCS (Hepes) and antibiotics (100 UI/ml penicillin, 25 µg/ml gentamycin). All culture dishes were from Nunc™.

*Production of adipose-derived tissue sheets.* Adipose-derived stromal cells were seeded at a density of 1.5 x10⁵ per well (1.58 x10⁴ /cm²) in 6-well plates containing an anchorage device allowing easy manipulation of the adipose sheets while preventing contraction. For the entire length of the experiments, the various media were supplemented with 50 µg/ml ascorbic acid (Sigma) which has been previously demonstrated to stimulate abundant extracellular matrix production from smooth muscle cells and dermal fibroblasts [L'Heureux N et al. FASEB Journal 1998;12:47-56, Michel M et al. In Vitro Cellular & Developmental Biology 1999; 35(6):318-326]. In order to produce adipocyte-filled cellular sheets, the stromal cultures were induced at selected times (7, 10, 14 or 21 days after seeding) by supplementing with an adipogenic cocktail (100 nM insuline, 0.2 nM T3, 1 µM dexamethasone, 0.25 mM IBMX and 1 µM rosiglitazone in 3% FCS for 3 days) while controls were treated in 3% FCS in standard medium. After 3 days, the induction medium was replaced with adipocyte medium (expansion medium with 100 nM insulin, 0.2 nM T₃ and 1 µM dexamethasone) while non-induced controls (conjunctive sheets) were cultured in 10% FCS expansion medium. Confluency was typically reached by day 3-4 after seeding. After 21-35 days depending on the cells population, manipulatable cellular sheets (3.5 cm² surface area) were ready to be assembled into thicker tissues.

*Production of reconstructed adipose tissue and reconstructed conjunctive tissue.* The reconstructed adipose tissue were produced by superimposing three adipose sheets (3.5 cm²) while their control counterparts were formed by superimposing three sheets of non-differentiated stromal cells. Typically, a strong cohesion between the different layers by culturing these issues for seven additional days in presence of ascorbic acid was observed.

*Production of a reconstructed skin tissues.* Adipose-derived human stromal cells were cultured in presence of ascorbic acid, induced or not to differentiate into adipocytes and combined with skin cells to produce new skin substitutes. Keratinocytes were seeded directly onto sheets of undifferentiated stromal cells or stromal cells differentiated for 12 days to form adipocytes. Alternatively, a tri-layered reconstructed skin was produced by seeding keratinocytes onto a stroma made of 2 cellular sheets of dermal fibroblasts layered on top of 2 cellular sheets of stromal cells, differentiated or not into adipocytes. The tissues were cultured immersed for 7 days and then raised at the air-liquid interface for 14 days for a total of 43 days in culture and 33 days of adipogenic differentiation.

*Quantification of adipose differentiation.* After 14 days of differentiation, independently of the time spent in culture before the time of adipogenic induction, Oil Red O (ORO) staining of the cytoplasmic neutral lipids of the droplets were performed according to a modification from [Kuri-Harcuch W et al. 1978;75(12):6107-9, Ramirez-Zacarias JL et al. Histochemistry 1992;97(6):493-7]. Briefly, cultures were rinsed, fixed with 10% buffered formalin, stained with 0.3% ORO in isopropanol and extracted with 4% Nonidet/isopropanol for quantification at 520 nm using a SpectraMax Plus^{™} spectrometer (Molecular Device) with SoftmaxPro^{™} Ver 4.7.1.

*Histological analysis.* At the indicated times, the reconstructed adipose tissue and reconstructed conjunctive tissue were processed for histological analysis. Tissue samples were harvested as 8 mm punch biopsy (Acuderm^{™}) and fixed in 10% buffered formalin before embedding in paraffin. 5 µm-thick tissue sections were stained for Masson's trichrome revealing collagens in blue. Lipids being extracted by organic solvents, the adipocyte lipid droplets are left as blank area on the histological sections. To further prove the presence of lipid-filled adipocytes within the reconstructed adipose tissue, samples were fixed with 10% formalin, embedded in OCT and 20 µm cryosections were stained with ORO. Photographs were taken on a Nikon^{™} Eclipse^{™} Ts100 microscope with a Nikon^{™} Coolpix^{™} 4500 camera.

*Scanning electron microscopy.* Samples of reconstructed adipose tissue and reconstructed conjunctive tissue were fixed with 2.5% glutaraldehyde in 0.1M cacodylate buffer for one hour before being processed either with hexamethyldisilazan or at the critical point followed by gold-palladium coating. All micrographs were obtained at 30 kV on a JEOL^{™} 6360LV SEM microscope (Tokyo, Japan).

*Adipokine secretion profiles.* Leptin and adiponectin secretion profiles were established by determining the adipokine concentrations in serum-free culture medium conditioned by the adipose-derived tissue and by the adipose tissue sheets for 24h using specific ELISA assays for human leptin (Bio Mol International, L.P.) and human adiponectin (R&D systems). Samples harvested from the cultures before induction of differentiation and each week thereafter were stored at -80°C/-20°C until analysis.

*Lipolysis measurements.* The lipolytic cell response of the adipose tissue sheets cultured for 27 days was assessed by measuring the amount of glycerol released into the incubation medium after treatment with 1 µM isoproterenol or its appropriate vehicle control. The tissues were previously incubated in serum-free medium for a minimum of 2 days to avoid interference with serum factors, and rinsed with serum-free medium before the assay. After 1, 2, 4 and 24 hours, the conditioned medium was immediately tested for the release of glycerol using an enzyme-coupled glycerol assay based on a colorimetric quantification (Sigma).

*Pro-angiogenic growth factor profiles.* Angiopoietin-1 and VEGF secretion profiles were established by determining the growth factor concentrations in serum-free culture medium conditioned by the adipose-derived tissue sheets and the adipose tissue sheets for 24h using specific ELISA assays for human angiopoietin-1 (R & D systems) and human VEGF (R & D systems). Samples harvested from the cultures before induction of differentiation and each week thereafter were stored at -80°C/-20°C until analysis.

### EXAMPLE II - Characterization of the reconstructed adipose tissue produced in Example I.

First, two modes of tissue harvesting were compared to determine if lipoaspiration procedures could be detrimental to the viability of stromal cells or affect their phenotype. Results are shown in Figure 1. Not only the yields obtained at the time of extraction were higher for LA cells (Fig. 1A), but the cells also proliferated slightly better in culture than LP cells (Fig. 1B). This implies that a mean of 500 000 stromal cells can be extracted per g of lipoaspirate, and considering that 400 g of tissue can easily be obtained and processed, 200 millions cells would be available for culturing, from which 2% could have stem cell characteristics [Strem BM et al. Trends Biotechnol 2005;23(2):64-6]. To our surprise, and as show in Figure 2, LA-denved cells also featured a higher adipogenic potential *in vitro* than the lipectomy-derived LP cells from excised fat. Moreover, the results indicate that extensive *in vivo* expansion of stromal cells, which is likely necessary for tissue engineering strategies, did not attenuate their adipogenic differentiation potential in presence of ascorbic acid, suggesting a good preservation of the progenitor cells under these culture conditions.

When cultured under appropriate conditions, namely ascorbic acid and adipogenic supplementation, manipulatable adipose sheets are obtained within 30 days. These adipose sheets are then assembled into thicker adipose tissues by superimposing multiple cellular sheets (Fig. 3), the latter were 139.4 ± 14.1 µm-thick, 3 layers, (n=7). Histologically, Masson's trichrome staining revealed that numerous adipocytes were embedded into a dense scaffold of extracellular matrix (Fig. 3). Finally, when view by SEM, the reconstructed adipose tissue were strikingly similar to human adipose tissue (Fig. 4).

Without wishing to be bound to theory, in the reconstructed adipose tissue described herein, adipose cells are building their own scaffold made of various matrix components that include collagens I, III and V, laminin-1 and high amounts of fibronectin.

Although cells extracted from lipectomy (LP) could be expanded and also generated 3D adipose sheets, they required longer culture time in order to produce a tissue easy to handle. Also, LP-generated cell sheets contained less adipocytes than LA-derived ones. Lipoaspirated fat is thus a very convenient source of cells that are performing well in our reconstructive strategy.

Adipogenic differentiation being a highly regulated process requiring cell confluency for adequate differentiation, it was investigated whether the dense matrix deposition occurring during the self-assembly phase would negatively impact on the ability to generate adipose substitutes. Figure 5 shows that inducing differentiation at either day 7, 10 or 14 of culture leads to a substantial differentiation into adipocytes after a fixed period of 14 days under adipogenic conditions. These results are particularly important because they reflect that reconstructed adipose tissue production parameters could be optimized to generate adipose tissues exhibiting a specific stage of differentiation, either young or fully differentiated.

Figure 6 also shows that ascorbic acid can promote adipocyte differentiation of stromal cells not submitted to freezing.

Figure 7 shows histological cross-sections of reconstructed human skin comprising adipose-derived stromal cells. In (A, B) only keratinocytes and stromal cells have been used whereas (C, D) keratinocytes, dermal fibroblasts and stromal cells have been used. Stromal cells found in (A, C) have not been induced with the adipogenic cocktail whereas stromal cells found in (B, D) have been induced with the adipogenic cocktail. Arrows in B and D point to lipid droplet accumulation within adipocytes. These results indicate that the adipose-derived stromal cells can be used to reconstruct *in vitro* the skin's hypoderm or serve as a dermal-like component.

Native adipose tissue has two major functions : storage energy as triglycerides and an important secretory function, liberating a variety of hormones, growth factors, cytokines and adipokines such as leptin and adiponectin. We investigated if the reconstructed adipose tissues displayed the biochemical characteristics of native adipocytes including adipokine secretion and response to β-adrenergic agonists. Both leptin (Fig. 8A) and adiponectin (Fig. 8B) were produced by reconstructed adipose tissue and increased with adipocyte differentiation. These results also indicate that human reconstructed adipose tissue are functional *in vitro* for at least 63 days in culture.

The functionality of mature human adipocytes embedded within the reconstructed adipose tissues to react appropriately to their environment was assessed by conducting lipolysis experiments (Fig. 9). Shown is one representative experiment of LA adipose tissue sheet cells in P3 containing adipocytes differentiated for 20 days. Expression is relative to basal levels at each time point. A peak in glycerol release 2h post-stimulation with 1 µm isoproterenol was observed. The lipolysis-mediated glycerol release (µM) measured indicates functionality of the adipocytes within the 3D adipose sheets. This suggests that upon grafting/implanting to a subject, the cells of the reconstructed adipose tissue would be responsive to their new environment.

The angiogenic potential of reconstructed conjunctive/adipose tissue was also evaluated by measuring the secretion of pro-angiogenic growth factors in the serum free supernatant of tissue cultures. As shown in Figure 10A, angiopoietin-1 is strongly expressed in reconstructed adipose tissue submitted to the induction cocktail when compared to reconstructed conjunctive tissue having non-induced adipose-derived stromal cells. On the other hand, VEGF seems to be more expressed in reconstructed adipose tissue not being induced with the adipogenic cocktail when compared with differentiated reconstructed adipose tissue (Figure 10B).

### EXAMPLE III - Production and characterization of vascularized reconstructed adipose tissue

Reconstructed adipose tissue and reconstructed conjunctive tissue were produced as outlined in Example I. After 20 days of culture, dermal endothelial cells have been added to three superimposed sheets and further cultured for 5 more days before, as outlined in Example I, superimposing the adipose sheets to form the vascularized reconstructed adipose/conjunctive tissue. Control reconstructed adipose tissue and reconstructed conjunctive tissue not seeded with endothelial cells have also been produced.

Results are shown in Figure 11. PECAM positive tubules have been observed in vascularized reconstructed adipose tissue and reconstructed conjunctive tissue only and not in control reconstructed adipose tissue or control reconstructed conjunctive tissue. In addition, among the tubular structures observed in those tissues, vascularized reconstructed adipose tissue induced with an adipogenic cocktail have more PECAM-specific tubular structures than vascularized reconstructed conjunctive tissue (22.5 ± 0.7%, n=2 cell lines) PECAM-expressing structures observed within reconstructed adipose tissue and reconstructed conjunctive tissue were more likely to show a distinct lumen reminiscent of a capillary. (A, D. F, H) are phase contrast micrographs corresponding to (A, C, E, F) respectively.

### EXAMPLE IV - Grafting of reconstructed adipose tissue unto athymic mice

Reconstructed adipose tissue containing four adipose tissue sheets produced as outlined in Example I have been grafted on the naked fascia of the posterior flank of an athymic mice. The area covered by the graft was measured by the ImageJ^{™} software. Fourteen days after grafting, the grafts looked healthy even though their thickness had increased and their surface area had decreased by 64% and 68% within 7 and 14 days respectively (Figure 12).

### EXAMPLE V - Genetic modification of isolated stromal cells

Monolayers of adipose-derived human stromal cells were produced as indicated in Example I. Cells were derived from a 51-year old subject. Three different cells types have then been submitted to genetic modification as described in Fradette et al. [Fradette et al. Gene Therapy 2005; 12: 48-58] using a MOI of 4: (i) human subconfluent stromal cells, (ii) human subconfluent stromal cells induced with the adipogenic cocktail for three days and (iii) mature adipocytes induced with the adipogenic cocktail for twenty days. The transgene inserted was recombinant human factor IX. Its expression level after transduction was measured with an ELISA assay (Cedarlane, ON). Results are shown in Figure 13 and indicate that the secretion of the paired antibodies is linked with the differentiation state of the cell and the number of days after infection. The phenomenon has also been observed for different transgenes (GDNF, NGF and IL-1Ra).

## Claims

1. A method of producing a manipulatable adipose-derived tissue sheet, said method comprising contacting isolated adipose-derived stromal cells with a first medium comprising ascorbic acid, thereby producing said manipulatable adipose-derived tissue sheet.

2. The method of claim 1, wherein the concentration of ascorbic acid in the first medium is from about 20 µg/ml to about 200 µg/ml, preferably about 50 µg/ml.

3. The method of claim 1, wherein said isolated adipose-derived stromal cells are derived from lipoaspirated fat or excised fat.

4. The method of claim 1, further comprising contacting said isolated adipose-derived stromal cells with a second medium comprising an adipogenic stimulus.

5. The method of claim 4, wherein said second medium comprises insulin, T3, dexamethasone, IBMX and a peroxisome proliferator-activated receptor gamma (PPARγ) agonist.

6. The method of claim 5, wherein said PPARγ agonist is rosiglitazone or pioglitazone.

7. The method of claim 1, wherein said manipulatable adipose-derived tissue sheet comprises adipose-derived stromal cells and wherein the concentration of said adipose-derived stromal cells in the manipulatable adipose-derived tissue sheet is preferably about 100%.

8. The method of claim 1, wherein said manipulatable adipose-derived tissue sheet comprises preadipocytes.

9. The method of claim 4, wherein said manipulatable adipose-derived tissue sheet comprises adipocytes and wherein the concentration of said adipocytes in the manipulatable adipose-derived tissue sheet is preferably between about 20 to 90%.

10. The method of claim 1, wherein said manipulatable adipose-derived tissue sheet comprises cells and an extracellular matrix and wherein said extracellular matrix is preferably produced by the adipose-derived stromal cells.

11. The method of claim 10, wherein the cells are mammalian cells, preferably human cells.

12. The method of claim 1 or 4, further comprising genetically modifying the adipose-derived stromal cells, wherein the genetic modification of the adipose-derived stromal cells is performed (i) prior to contacting the adipose-derived stromal cells with said first medium, (ii) simultaneously to contacting the adipose-derived stromal cells with said first medium, (iii) after contacting the adipose-derived stromal cells with said first medium.

13. The method of claim 1 or 4, further comprising adding a further cell type to the manipulatable adipose-derived tissue sheet and wherein the further cell type is preferably at least one of an endothelial cell type, an epithelial cell type, a fibroblastic cell type, a muscular cell type and a neuronal cell type.

14. A method of producing a reconstructed conjunctive tissue, said method comprising:
producing at least two manipulatable adipose-derived tissue sheets according to the method of claim 1; and
superimposing said at least two manipulatable adipose-derived tissue sheets, thereby producing the reconstructed conjunctive tissue.

15. A method of producing a reconstructed adipose tissue, said method comprising:
producing at least two manipulatable adipose-derived tissue sheets according to the method of claim 4; and
superimposing said at least two manipulatable adipose-derived tissue sheets, thereby producing the reconstructed adipose tissue.

16. A method of determining the adipocyte-modulating properties of an agent, said method comprising:
(i) producing a manipulatable adipose-derived tissue sheet according to the method of claim 4, or producing a reconstructed adipose tissue according to the method of claim 15;
(ii) contacting said agent with said tissue; and
(iii) determining if said contact modulates a parameter of said tissue, thereby indicating the adipocyte-modulating properties of said agent.

17. The method of claim 16, wherein said parameter of said tissue is at least one of the number of cells in the tissue, the size of the cells in the tissue, the roundness of the cells in the tissue, the degree of differentiation of the cells in the tissue, the quantity of lipids in the cells of the tissue, the composition of lipids in the cells of the tissue, the ability of the cells of the tissue to produce an adipokine, the ability of the cells of the tissue to produce a pro-angiogenic growth factor, the ability of the cells of the tissue to respond to a lipolytic stimuli, the ability of the cells of the tissue to replicate, the viability of the cells of the tissue and the ability of the cells of the tissue to express a gene related to an adipocyte metabolic function.

18. The method of claim 17, wherein said gene is at least one of FOXC2, PGC-1, UCP-1, GATA2, PPARα and PPARγ.

## Patentansprüche

1. Verfahren zur Herstellung einer manipulierbaren von Fett abgeleiteten Gewebeschicht, das Verfahren umfassend Kontaktieren von isolierten von Fett abgeleiteten Stromazellen mit einem ersten Medium, das Ascorbinsäure umfasst, wodurch die manipulierbare von Fett abgeleitete Gewebeschicht produziert wird.

2. Verfahren nach Anspruch 1, wobei die Konzentration der Ascorbinsäure im ersten Medium von etwa 20 µg/ml bis etwa 200 µg/ml, vorzugsweise etwa 50 µg/ml beträgt.

3. Verfahren nach Anspruch 1, wobei die isolierten von Fettabgeleiteten Stromazellen von Fett aus Lipoaspiraten oder ausgeschnittenem Fett abgeleitet sind.

4. Verfahren nach Anspruch 1, ferner umfassend Kontaktieren der isolierten von Fett abgeleiteten Stromazellen mit einem zweiten Medium, das einen adipogenen Stimulus umfasst.

5. Verfahren nach Anspruch 4, wobei das zweite Medium Insulin, T3, Dexamethason, IBMX und einen Peroxisom-Proliferator-aktivierter-Rezeptor-gamma (PPARγ)-Agonisten umfasst.

6. Verfahren nach Anspruch 5, wobei der PPARγ-Agonist Rosiglitazon oder Pioglitazon ist.

7. Verfahren nach Anspruch 1, wobei die manipulierbare Fett-abgeleitete Gewebeschicht von Fett abgeleitete Stromazellen umfasst und wobei die Konzentration der von Fett abgeleiteten Stromazellen in der manipulierbaren von Fett abgeleiteten Gewebeschicht vorzugsweise etwa 100 % beträgt.

8. Verfahren nach Anspruch 1, wobei die manipulierbare von Fett abgeleitete Gewebeschicht Präadipozyten umfasst.

9. Verfahren nach Anspruch 4, wobei die manipulierbare von Fett abgeleitete Gewebeschicht Adipozyten umfasst und wobei die Konzentration der Adipozyten in der manipulierbaren von Fett abgeleiteten Gewebeschicht vorzugsweise zwischen etwa 20 bis 90 % beträgt.

10. Verfahren nach Anspruch 1, wobei die manipulierbare von Fett abgeleitete Gewebeschicht Zellen und eine extrazelluläre Matrix umfasst, und wobei die extrazelluläre Matrix vorzugsweise durch die von Fett abgeleiteten Stromazellen produziert wird.

11. Verfahren nach Anspruch 10, wobei die Zellen Säugerzellen sind, vorzugsweise menschliche Zellen.

12. Verfahren nach Anspruch 1 oder 4, ferner umfassend genetisch Modifizieren der von Fett abgeleiteten Stromazellen, wobei die genetische Modifizierung der von Fettabgeleiteten Stromazellen (i) vor dem Kontaktieren der von Fettabgeleiteten Stromazellen mit dem ersten Medium, (ii) gleichzeitig mit Kontaktieren der von Fett abgeleiteten Stromazellen mit dem ersten Medium, (iii) nach Kontaktieren der von Fett abgeleiteten Stromazellen mit dem ersten Medium durchgeführt wird.

13. Verfahren nach Anspruch 1 oder 4, ferner umfassend Zugeben eines weiteren Zelltyps zu der manipulierbaren von Fett abgeleiteten Gewebeschicht und wobei der weitere Zelltyp vorzugsweise mindestens einer von einem endothelialen Zelltyp, einem epithelialen Zelltyp, einem Fibroblasten-Zelltyp, einem Muskel-Zelltyp und einem neuronalen Zelltyp ist.

14. Verfahren zum Produzieren eines rekonstruierten Bindegewebes, das Verfahren umfassend:
Produzieren von mindestens zwei manipulierbaren von Fett abgeleiteten Gewebeschichten nach dem Verfahren von Anspruch 1; und
Überlagern der mindestens zwei manipulierbaren von Fett abgeleiteten Gewebeschichten, wodurch das rekonstruierte Bindegewebe produziert wird.

15. Verfahren zum Produzieren eines rekonstruierten Fettgewebes, das Verfahren umfassend:
Produzieren von mindestens zwei manipulierbaren von Fett abgeleiteten Gewebeschichten nach dem Verfahren von Anspruch 4; und
Überlagern der mindestens zwei manipulierbaren von Fett abgeleiteten Gewebeschichten, wodurch das rekonstruierte Fettgewebe produziert wird.

16. Verfahren zur Bestimmung der Adipozyten-modulierenden Eigenschaften eines Mittels, das Verfahren umfassend:
(i) Produzieren einer manipulierbaren von Fett abgeleiteten Gewebeschicht nach dem Verfahren von Anspruch 4 oder Produzieren eines rekonstruierten Fettgewebes nach dem Verfahren von Anspruch 15;
(ii) Kontaktieren des Mittels mit dem Gewebe; und
(iii) Bestimmen, ob der Kontakt einen Parameter des Gewebes moduliert, wodurch die Adipozyten-modulierenden Eigenschaften des Mittels angezeigt werden.

17. Verfahren nach Anspruch 16, wobei der Parameter des Gewebes mindestens einer von der Anzahl der Zellen in dem Gewebe, der Größe der Zellen in dem Gewebe, der Rundheit der Zellen in dem Gewebe, dem Grad der Differenzierung der Zellen in dem Gewebe, der Menge an Lipiden in den Zellen des Gewebes, der Zusammensetzung von Lipiden in den Zellen des Gewebes, der Fähigkeit der Zellen des Gewebes, ein Adipokin zu produzieren, der Fähigkeit der Zellen des Gewebes, einen pro-angiogenen Wachstumsfaktor zu produzieren, der Fähigkeit der Zellen des Gewebes auf lipolytische Stimuli zu antworten, der Fähigkeit der Zellen des Gewebes zu replizieren, der Lebensfähigkeit der Zellen des Gewebes und der Fähigkeit der Zellen des Gewebes, ein Gen in Bezug auf eine Adipozyten-Stoffwechselfunktion zu exprimieren, ist.

18. Verfahren nach Anspruch 17, wobei das Gen mindestens eines von FOXC2, PGC-1, UCP-1, GATA2, PPARα und PPARγ ist.

## Revendications

1. Procédé de production d'une couche de tissu d'origine adipeuse manipulable, ledit procédé comprenant la mise en contact de cellules stromales d'origine adipeuse isolées avec un premier milieu comprenant de l'acide ascorbique, produisant ainsi ladite couche de tissu d'origine adipeuse manipulable.

2. Procédé selon la revendication 1, dans lequel la concentration d'acide ascorbique dans le premier milieu est d'environ 20 µg/ml à environ 200 µg/ml, de préférence d'environ 50 µg/ml.

3. Procédé selon la revendication 1, dans lequel lesdites cellules stromales d'origine adipeuse isolées sont issues de graisse extraite par lipoaspiration ou excision.

4. Procédé selon la revendication 1, comprenant en outre la mise en contact desdites cellules stromales d'origine adipeuse isolées avec un deuxième milieu comprenant un stimulus adipogène.

5. Procédé selon la revendication 4, dans lequel ledit deuxième milieu comprend de l'insuline, de la T3, de la dexaméthasone, de l'IBMX et un agoniste des récepteurs gamma activés par les proliférateurs de peroxysomes (PPAR gamma).

6. Procédé selon la revendication 5, dans lequel ledit agoniste des PPAR gamma est la rosiglitazone ou la pioglitazone.

7. Procédé selon la revendication 1, dans lequel ladite couche de tissu d'origine adipeuse manipulable comprend des cellules stromales d'origine adipeuse et dans lequel la concentration desdites cellules stromales d'origine adipeuse dans la couche de tissu d'origine adipeuse manipulable est, de préférence, d'environ 100 %.

8. Procédé selon la revendication 1, dans lequel ladite couche de tissu d'origine adipeuse manipulable comprend des préadipocytes.

9. Procédé selon la revendication 4, dans lequel ladite couche de tissu d'origine adipeuse manipulable comprend des adipocytes et dans lequel la concentration desdits adipocytes dans la couche de tissu d'origine adipeuse manipulable est comprise, de préférence, entre 20 % et 90 % environ.

10. Procédé selon la revendication 1, dans lequel ladite couche de tissu d'origine adipeuse manipulable comprend des cellules et une matrice extracellulaire et dans lequel ladite matrice extracellulaire est produite de préférence par les cellules stromales d'origine adipeuse.

11. Procédé selon la revendication 10, dans lequel les cellules sont des cellules de mammifère, de préférence des cellules humaines.

12. Procédé selon la revendication 1 ou la revendication 4, comprenant en outre la modification génétique des cellules stromales d'origine adipeuse et dans lequel la modification génétique des cellules stromales d'origine adipeuse est réalisée i) avant la mise en contact des cellules stromales d'origine adipeuse avec ledit premier milieu, ii) simultanément à la mise en contact des cellules stromales d'origine adipeuse avec ledit premier milieu, iii) après la mise en contact des cellules stromales d'origine adipeuse avec ledit premier milieu.

13. Procédé selon la revendication 1 ou la revendication 4, comprenant en outre l'ajout d'un autre type de cellule à la couche de tissu d'origine adipeuse manipulable et dans lequel l'autre type de cellule est, de préférence, au moins un type parmi un type de cellules endothéliales, un type de cellules épithéliales, un type de cellules fibroblastiques, un type de cellules musculaires et un type de cellules neuronales.

14. Procédé de production d'un tissu conjonctif reconstruit, ledit procédé comprenant :
la production d'au moins deux couches de tissu d'origine adipeuse manipulables selon le procédé de la revendication 1 ; et
la superposition desdites au moins deux couches de tissu d'origine adipeuse manipulables, produisant ainsi le tissu conjonctif reconstruit.

15. Procédé de production d'un tissu adipeux reconstruit, ledit procédé comprenant :
la production d'au moins deux couches de tissu d'origine adipeuse manipulables selon le procédé de la revendication 4 ; et
la superposition desdites au moins deux couches de tissu d'origine adipeuse manipulables, produisant ainsi le tissu adipeux reconstruit.

16. Procédé de détermination des propriétés de modulation des adipocytes d'un agent, ledit procédé comprenant :
i) la production d'une couche de tissu d'origine adipeuse manipulable selon le procédé de la revendication 4, ou produire un tissu adipeux reconstruit selon le procédé de la revendication 15 ;
ii) la mise en contact dudit agent avec ledit tissu ; et
iii) la détermination du fait que ledit contact module un paramètre dudit tissu, indiquant de ce fait les propriétés de modulation des adipocytes dudit agent.

17. Procédé selon la revendication 16, dans lequel ledit paramètre dudit tissu est au moins un paramètre parmi le nombre de cellules dans le tissu, la taille des cellules dans le tissu, la rondeur des cellules dans le tissu, le degré de différenciation des cellules dans le tissu, la quantité de lipides dans les cellules du tissu, la composition de lipides dans les cellules du tissu, la capacité des cellules du tissu à produire une adipokine, la capacité des cellules du tissu à produire un facteur de croissance proangiogénique, la capacité des cellules du tissu à réagir à un stimulus lipolytique, la capacité des cellules du tissu à se reproduire, la viabilité des cellules du tissu et la capacité des cellules du tissu à exprimer un gène lié à une fonction métabolique adipocyte.

18. Procédé selon la revendication 17, dans lequel ledit gène est au moins un gène parmi FOXC2, PGC-1, UCP-1, GATA2, PPAR alpha et PPAR gamma.
